(19) **Europäisches Patentamt European Patent Office Office européen des brevets**

(11) **EP 4 425 175 B1**

(12) **EUROPEAN PATENT SPECIFICATION**

(45) Date of publication and mention of the grant of the patent:
**11.02.2026 Bulletin 2026/07**

(21) Application number: **23878716.2**

(22) Date of filing: **06.07.2023**

(51) International Patent Classification (IPC):
*G01N 33/00* (2006.01)      *G06F 17/00* (2019.01)
*G06F 30/20* (2020.01)      *G06F 30/25* (2020.01)
*G16C 60/00* (2019.01)

(52) Cooperative Patent Classification (CPC):
**G06F 30/20; G06F 30/25; G16C 60/00;**
G06F 2111/10; G16C 20/10; Y02P 90/30

(86) International application number:
**PCT/CN2023/106203**

(87) International publication number:
**WO 2024/082719 (25.04.2024 Gazette 2024/17)**

(54) **METHOD FOR PREDICTING CRITICAL CAKING PERIOD OF CRYSTAL PARTICLE**

VERFAHREN ZUR VORHERSAGE DER KRITISCHEN VERBACKUNGSPERIODE VON KRISTALLPARTIKELN

PROCÉDÉ DE PRÉDICTION DE PÉRIODE CRITIQUE D'AGGLOMÉRATION DE PARTICULES CRISTALLINES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **20.10.2022 CN 202211289850**

(43) Date of publication of application:
**04.09.2024 Bulletin 2024/36**

(73) Proprietors:
• **Tianjin University**
  **Tianjin 300072 (CN)**
• **Zhejiang Huakang Pharmaceutical Co., Ltd.**
  **Quzhou, Zhejiang 324302 (CN)**
• **Zhejiang Institute of Tianjin University (Shaoxing)**
  **Shaoxing, Zhejiang 312300 (CN)**

(72) Inventors:
• **GONG, Junbo**
  **Tianjin 300072 (CN)**
• **LI, Mian**
  **Santa Clara, CA 95054 (US)**
• **CHEN, Mingyang**
  **Tianjin 300072 (CN)**
• **YANG, Wulong**
  **Quzhou, Zhejiang 324302 (CN)**
• **LI, Jiahui**
  **Tianjin 300072 (CN)**
• **LI, Mingxuan**
  **Tianjin 300072 (CN)**
• **WU, Qiang**
  **Quzhou, Zhejiang 324302 (CN)**
• **HOU, Baohong**
  **Tianjin 300072 (CN)**
• **YIN, Qiuxiang**
  **Tianjin 300072 (CN)**

(74) Representative: **Sun, Yiming**
  **HUASUN Patent- und Rechtsanwälte**
  **Friedrichstraße 33**
  **80801 München (DE)**

(56) References cited:
CN-A- 105 784 480      CN-A- 107 063 906
CN-A- 108 959 817      CN-A- 108 959 817
CN-A- 114 965 186      CN-A- 115 684 484
JP-A- 2004 157 086      US-A1- 2007 105 226

- CHEN MINGYANG ET AL: "The time and location dependent prediction of crystal caking by a modified crystal bridge growth model and DEM simulation considering particle size and shape", CHEMICAL ENGINEERING SCIENCE, OXFORD, GB, vol. 214, 9 December 2019 (2019-12-09), XP086046965, ISSN: 0009-2509, [retrieved on 20191209], DOI: 10.1016/J.CES.2019.115419
- WANG QING;: "Predictive model for hygroscopicity of contents in Guizhi Fuling Capsules", CHINA JOURNAL OF CHINESE MATERIA MEDICA, vol. 45, no. 2, 28 February 2020 (2020-02-28), pages 242 - 249, XP093160142
- ZENG LING-PING: "Study on the hygroscopicity mechanism and measurement method of the granulated seasonings", CHINA CONDIMENT, vol. 34, no. 4, 10 April 2009 (2009-04-10), pages 85 - 89, XP093160146
- LI YANHUA: "Research Progress on the Interactions Among Particles and Caking Behavior of Food Powder ", JOURNAL OF THE CHINESE CEREALS AND OILS ASSOCIATION, vol. 34, no. 3, 15 March 2019 (2019-03-15), pages 126 - 132, XP093160144

**Description**

**TECHNICAL FIELD**

The present disclosure relates to a technology field of agglomeration of crystal particles, and in particular relates to a method for predicting a critical agglomeration cycle of a crystal particle.

**BACKGROUND**

The agglomeration of crystal particles refers to a process in which the crystal particles with good fluidity and dispersibility originally, aggregate with each other to form an irregular agglomerate. An agglomeration phenomenon of the crystal particles is easy to occur in a post-processing (i.e., a transportation, storage, and drying of the crystal particles) for crystal particle products, resulting in degradation of performance of the crystal particle products. Therefore, operations of determining critical agglomeration cycles of the crystal particle under different temperature and humidity storage conditions and realizing a prediction of the critical agglomeration cycle are crucial for guiding growth, storage, and transportation of the crystal particle products.

However, the prediction of the critical agglomeration cycle of the crystal particle is realized mainly based on a correspondence relationship between an accelerated agglomeration experimental process under a high temperature and high humidity in the laboratory and an agglomeration rate under an actual storage environment, which results in a long experimental period (e.g., months or even a year) and a large experimental quantity, and a single experiment can only determine a critical agglomeration cycle under a specific humidity cycle condition, which can not achieve fast and accurate prediction for particle groups under different temperature and humidity conditions. In addition, the critical agglomeration cycle of the crystal particle shows a high correlation with a size of the crystal particle, and the current general knowledge is that the larger the size of the crystal particle, the longer the critical agglomeration cycle is, while a quantitative mathematical relationship has not been established to realize a theoretical guidance of the agglomeration process yet.

Therefore, it is desired to provide a method for predicting the critical agglomeration cycle of the crystal particle, to realize fast and accurate prediction of the critical agglomeration cycle of the crystal particle product with small experimental quantity and low overall cost.

**SUMMARY**

The technical problem solved by the present invention is to provide a method for predicting the critical agglomeration cycle of the crystal particle to overcome defects of long testing time, large measurement workload, difficult prediction, and high cost of the critical agglomeration cycles for existed crystal particle products, which can realize fast and accurate prediction of the critical agglomeration cycle of the crystal particle product with small experimental quantity and low overall cost and satisfy actual growth needs.

The present disclosure is realized by providing method for predicting a critical agglomeration cycle of a crystal particle, wherein the method is executed by a processor, the method comprises: establishing a CHS crystal bridge growth model database of a crystal particle with a same type of a crystal particle to be predicted firstly, wherein the CHS crystal bridge growth model database includes: data of equivalent particle radius corresponding to different particle size standards of the same type of crystal particle, data of a moisture absorption capacity of crystal particles with different equivalent particle radiuses under multiple ambient temperatures and multiple ambient humidity conditions, respectively, and data of critical agglomeration cycles of the crystal particles with different equivalent particle radiuses under the multiple ambient temperatures and multiple environmental high and low humidity cycle conditions, respectively; then selecting existed data in the corresponding CHS crystal bridge growth model database based on an equivalent particle radius, a stored ambient temperature, and an environmental high and low humidity cycle condition of the crystal particle to be predicted, respectively, and calculating the critical agglomeration cycle according to following calculation equations, wherein a result obtained by calculation is a predicted critical agglomeration cycle of the crystal particle to be predicted;

the calculation equations include:

(1) in response to an ambient temperature and an environmental high and low humidity cycle condition of the crystal particle to be predicted being the same with data in the CHS crystal bridge growth model database and merely the equivalent particle radius of the crystal particle to be predicted being different from data in the CHS crystal bridge growth model database, a critical agglomeration cycle $N_1'$ of the crystal particle to be predicted under the ambient temperature and the environmental high and low humidity cycle condition according to equation (a):

$$\frac{N_1}{N_1'} = \left(\frac{R_{1e}}{R_{1e}'}\right)^4, \text{ equation (a)}$$

in the above equation, $R_{1e}$ represents an equivalent particle radius of an existed crystal particle in the CHS crystal bridge growth model database, $R_{1e}'$ represents the equivalent particle radius of the crystal particle to be predicted, $N_1$ represents a critical agglomeration cycle of existed crystal particles with equivalent particle radius $R_{1e}$ in the CHS crystal bridge growth model under the ambient temperature and the environmental high and low humidity cycle condition, $N_1'$ represents the critical agglomeration cycle of the crystal particle to be predicted, and $N_1$ and $N_1'$ are an integer not less than 1 , respectively;

(2) in response to the ambient temperature and the equivalent particle radius of the crystal particle to be predicted are the same with the data in the CHS crystal bridge growth model database and merely the environmental high and low humidity cycle condition of the crystal particle to be predicted being different from data in the CHS crystal bridge growth model database, a critical agglomeration cycle $N_2''$ of the crystal particle to be predicted under the ambient temperature and the environmental high and low humidity cycle condition according to equation (b):

$$\frac{N_2}{N_2''} = \frac{V_l'}{V_l}, \text{ equation (b)}$$

wherein,

$$V_l = V_{RH2} - V_{RH1}, \text{ equation (c)};$$

$$V_l' = V_{RH3} - V_{RH1}, \text{ equation (d)};$$

in the above equations, $V_{RH2}$ represents a moisture absorption capacity of the existed crystal particle in the CHS crystal bridge growth model database under a high humidity condition in a first environmental high and low humidity cycle; $V_{RH3}$ represents a moisture absorption capacity of the existed crystal particle in the CHS crystal bridge growth model database under a high humidity condition in a second environmental high and low humidity cycle, wherein a low humidity condition in the first environmental high and low humidity cycle is the same with a low humidity condition in the second environmental high and low humidity cycle; $V_{RH1}$ represents a moisture absorption capacity of the existed crystal particle in the CHS crystal bridge growth model database under a low humidity condition in the first environmental high and low humidity cycle; $V_l$ represents a moisture absorption capacity difference between the high humidity condition and the low humidity condition in the first environmental high and low humidity cycle; $V_l'$ represents a moisture absorption capacity difference between the high humidity condition and the low humidity condition in the second environmental high and low humidity cycle; $N_2$ represents a critical agglomeration cycle of the existed crystal particle in the CHS crystal bridge growth model database under the high humidity condition and the low humidity condition in the first environmental high and low humidity cycle; $N_2''$ represents a critical agglomeration cycle of the crystal particle to be predicted under the high humidity condition and the low humidity condition in the second environmental high and low humidity cycle, and $N_2$ and $N_2''$ are an integer not less than 1, respectively;

wherein the high humidity condition in the environmental high and low humidity cycle is lower than a deliquescence point of the same type of crystal particles, such that the critical agglomeration cycle is greater than one when a particle size of the crystal particle is less than 100 microns, the low humidity condition is less than the high humidity condition, and a humidity difference between the low humidity condition and the high humidity condition is greater than 20%.

Further, the equivalent particle radius of the crystal particle and the equivalent particle radius of the crystal particle to be predicted in the CHS crystal bridge growth model database are obtained by following operations:
weighing a mass of each particle size interval of the crystal particles after performing particle size screening on crystal particles to be measured, obtaining data $m_1, m_2, ..., m_n$, sequentially, counting a count of crystal particles for each particle size interval, obtaining data $P_1, P_2, ..., P_n$, sequentially, and calculating an equivalent particle radius $R_e$ of the crystal particle to be predicted according to following equations (e) and (f):

$$P_1 R_1^3 + P_2 R_2^3 + \cdots + P_n R_n^3 = (P_1 + P_2 + \cdots + P_n) R_e^3 , \qquad \text{equation (e)};$$

$$P_1 R_1{}^3 : P_2 R_2{}^3 : \ldots : P_n R_n{}^3 = m_1 : m_2 : \ldots : m_n \, , \qquad \text{equation (f)}$$

Further, the critical agglomeration cycle of the crystal particles with different equivalent particle radiuses under the multiple ambient temperatures and multiple environmental high and low humidity cycle conditions is obtained by: loading a crystal particle to be measured into an agglomeration mold and placing the agglomeration mold in a box with set temperature, setting a high humidity and low humidity in a high and low humidity cycle within the both being the same with a high and low humidity cycle of the crystal particles to be measured, wherein a period of the high and low humidity cycle is 12-24 hours; counting a time of the high and low humidity cycle, and performing a de-molding operation for an agglomerate in the agglomeration mold; during the de-molding operation, in response to the agglomerate falls apart, determining that the agglomerate of the crystal particle to be measured is not agglomerated, and continuing to perform above operations to a next cycle until the agglomerate of the crystal particle does not fall apart, and recording a count of cycles completed to prevent the agglomeration from falling apart, and determining the count of cycles as a critical agglomeration cycle of the crystal particle to be measured under a corresponding ambient temperature and environmental high and low humidity cycle condition.

Further, when determining the critical agglomeration cycle, if the critical agglomeration cycle repeatedly determines for three times, calculating an average of the reference critical agglomeration cycles determined by three times as a critical agglomeration cycle after averaging treatment.

Further, $V_{RH1}$, $V_{RH2}$, and $V_{RH3}$ in the equation (c) and equation (d) are calculated by:

under a same ambient temperature, a same environmental humidity, and different equivalent particle radiuses, calculating a moisture absorption capacity $V_{RH}'$ of a crystal particle with an equivalent particle radius $R_e'$ under an environmental humidity condition RH based on following equation (g):

$$\frac{V_{RH}}{V_{RH}{}'} = \frac{R_e}{R_e{}'}, \text{ equation (g)}$$

wherein $V_{RH}$ represents a moisture absorption capacity of existed crystal particles with the equivalent particle radius $R_e$ under the environmental humidity condition RH in the CHS crystal bridge growth model database, and $V_{RH}'$ is one of $V_{RH1}$, $V_{RH2}$, and $V_{RH3}$.

Further, a determination of the moisture absorption capacity is performed by a dynamic steam adsorption instrument, before the determination, performing an initial drying treatment for the crystal particle to be measured in advance, and a mass of the crystal particle to be measured does not exceed 10 mg.

Further, the crystal particle includes a xylitol crystal particle, and a high humidity condition of the environmental high and low humidity cycle for the xylitol crystal particle is 65% and a low humidity condition of the environmental high and low humidity cycle for the xylitol crystal particle is 30%.

Prior art article by CHEN MINGYANG ET AL ("The time and location dependent prediction of crystal caking by a modified crystal bridge growth model and DEM simulation considering particle size and shape",CHEMICAL ENGINEERING SCIENCE, OXFORD, GB, vol. 214, 9 December 2019 (2019-12-09), ISSN: 0009-2509, DOI: 10.1016/ J.CES.2019.115419) defines a method for prediction of caking using DEM-simulation and physical model. CN 108 959 817 A is another prior art document using analytical or empirical evaluation of caking strength under different humidity conditions. Compared with prior art, the method for predicting the critical agglomeration cycle of the crystal particle in the present disclosure includes following operations: stablishing a CHS crystal bridge growth model database of a crystal particle with a same type of a crystal particle to be predicted firstly, then selecting existed data in the corresponding CHS crystal bridge growth model database based on an equivalent particle radius, a stored ambient temperature, and an environmental high and low humidity cycle condition of the crystal particle to be predicted, respectively, and calculating the critical agglomeration cycle according to an experience calculation equation, wherein a result obtained by calculation is a predicted critical agglomeration cycle of the crystal particle to be predicted. The present disclosure has characteristics of time-saving, convenience, good universality, and high prediction accuracy, which can quickly predict the critical agglomeration cycle of multi-particle crystal particle products under different humidity storage conditions within a week, resulting in greatly reducing time costs and providing guidance for storage of industrial crystal particle products.

**BRIEF DESCRIPTION OF THE DRAWINGS**

FIG. 1 is a schematic diagram illustrating a structure profile of an agglomeration mold used for determining a critical agglomeration cycle according to some embodiments of the present disclosure;

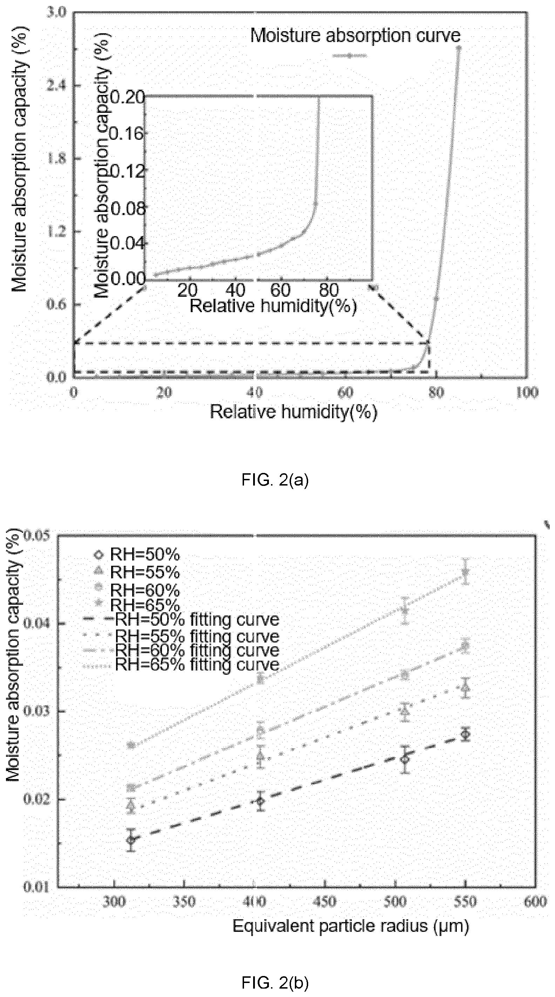

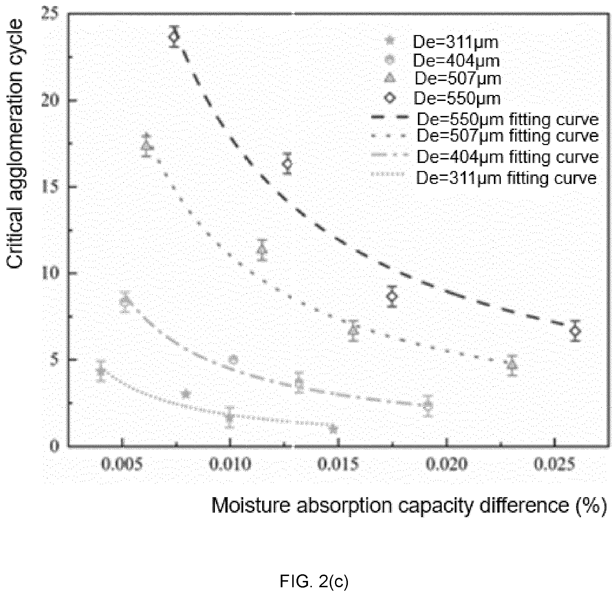

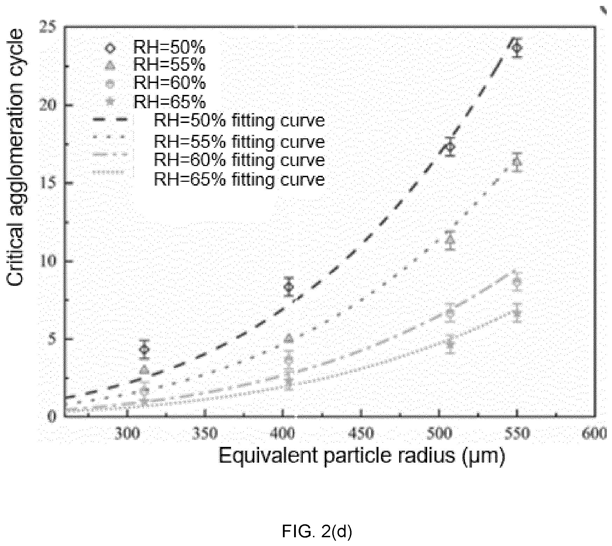

FIG. 2 is a schematic diagram illustrating a comparison between a measured result and a prediceted result obtained by a CHS crystal bridge growth model database and a moisture absorption document of a xylitol crystal particle product in an embodiment 1, wherein (a) is a moisture absorption curve of the xylitol crystal particle at a temperature of 25 °C, (b) is a comparison diagram between a measured result and a predicted result of a moisture absorption capacity and an equivalent particle radius of the xylitol crystal particle, (c) is a comparison diagram between a measured result and a predicted result of a critical agglomeration cycle and a moisture absorption capacity difference of the xylitol crystal particle, and (d) is a comparison diagram between a measured result and a predicted result of a critical agglomeration cycle and an equivalent particle radius of the xylitol crystal particle;

FIG. 3 is a schematic diagram illustrating a fitting of predicted results of critical agglomeration cycles of a multi-particle crystal product of a xylitol crystal particle in embodiment 1 under different humidity storage conditions according to some embodiments of the present disclosure.

## DETAILED DESCRIPTION

In order to make the objects, technical solutions, and advantages of the present disclosure clearer and more understandable, the present disclosure is described in further detail hereinafter in conjunction with the accompanying drawings and embodiments. It should be understood that the specific embodiments described herein are only for explaining the present disclosure and are not intended to limit the present disclosure.

The present disclosure provides a relatively great embodiment of the method for predicting a critical agglomeration cycle of a crystal particle, the method may include following operation: establishing a CHS crystal bridge growth model database of a crystal particle with a same type of a crystal particle to be predicted firstly, wherein the CHS crystal bridge growth model database includes: data of equivalent particle radius corresponding to different particle size standards of the same type of crystal particle, data of a moisture absorption capacity of crystal particles with different equivalent particle radiuses under multiple ambient temperatures and multiple ambient humidity conditions, respectively, and data of critical agglomeration cycles of the crystal particles with different equivalent particle radiuses under the multiple ambient temperatures and multiple environmental high and low humidity cycle conditions, respectively; then selecting existed data in the corresponding CHS crystal bridge growth model database based on an equivalent particle radius, a stored ambient temperature, and an environmental high and low humidity cycle condition of the crystal particle to be predicted, respectively, and calculating the critical agglomeration cycle according to following calculation equations, wherein a result obtained by calculation is a predicted critical agglomeration cycle of the crystal particle to be predicted. the calculation equations include:

(1) in response to an ambient temperature and an environmental high and low humidity cycle condition of the crystal particle to be predicted being the same with data in the CHS crystal bridge growth model database and merely the equivalent particle radius of the crystal particle to be predicted being different from data in the CHS crystal bridge growth model database, a critical agglomeration cycle $N_1'$ of the crystal particle to be predicted under the ambient temperature and the environmental high and low humidity cycle condition according to equation (a):

$$\frac{N_1}{N_1'} = \left(\frac{R_{1e}}{R_{1e}'}\right)^4, \text{ equation (a)}$$

in the above equation, $R_{1e}$ represents an equivalent particle radius of an existed crystal particle in the CHS crystal bridge growth model database, $R_{1e}'$ represents the equivalent particle radius of the crystal particle to be predicted, $N_1$ represents a critical agglomeration cycle of existed crystal particles with equivalent particle radius $R_{1e}$ in the CHS crystal bridge growth model under the ambient temperature and the environmental high and low humidity cycle condition, $N_1'$ represents the critical agglomeration cycle of the crystal particle to be predicted, and $N_1$ and $N_1'$ are an integer not less than 1 , respectively;

(2) in response to the ambient temperature and the equivalent particle radius of the crystal particle to be predicted are the same with the data in the CHS crystal bridge growth model database and merely the environmental high and low humidity cycle condition of the crystal particle to be predicted being different from data in the CHS crystal bridge growth model database, a critical agglomeration cycle $N_2''$ of the crystal particle to be predicted under the ambient temperature and the environmental high and low humidity cycle condition according to equation (b):

$$\frac{N_2}{N_2{''}} = \frac{V_l{'}}{V_l}, \text{ equation (b)}$$

wherein,

$$V_l = V_{RH2} - V_{RH1}, \text{ equation (c)};$$

$$V_l{'} = V_{RH3} - V_{RH1}, \text{ equation (d)};$$

in the above equations, $V_{RH2}$ represents a moisture absorption capacity of the existed crystal particle in the CHS crystal bridge growth model database under a high humidity condition in a first environmental high and low humidity cycle; $V_{RH3}$ represents a moisture absorption capacity of the existed crystal particle in the CHS crystal bridge growth model database under a high humidity condition in a second environmental high and low humidity cycle, wherein a low humidity condition in the first environmental high and low humidity cycle is the same with a low humidity condition in the second environmental high and low humidity cycle; $V_{RH1}$ represents a moisture absorption capacity of the existed crystal particle in the CHS crystal bridge growth model database under a low humidity condition in the first environmental high and low humidity cycle; $V_l$ represents a moisture absorption capacity difference between the high humidity condition and the low humidity condition in the first environmental high and low humidity cycle; $V_l{'}$ represents a moisture absorption capacity difference between the high humidity condition and the low humidity condition in the second environmental high and low humidity cycle; $N_2$ represents a critical agglomeration cycle of the existed crystal particle in the CHS crystal bridge growth model database under the high humidity condition and the low humidity condition in the first environmental high and low humidity cycle; $N_2{''}$ represents a critical agglomeration cycle of the crystal particle to be predicted under the high humidity condition and the low humidity condition in the second environmental high and low humidity cycle, and $N_2$ and $N_2{''}$ are an integer not less than 1, respectively;

wherein the high humidity condition in the environmental high and low humidity cycle is lower than a deliquescence point of the same type of crystal particles, such that the critical agglomeration cycle is greater than one when a particle size of the crystal particle is less than 100 microns, the low humidity condition is less than the high humidity condition, and a humidity difference between the low humidity condition and the high humidity condition is greater than 20%. For example, if the deliquescence point of the xylitol crystal particle is 75%, the most suitable high humidity condition of the high and low humidity cycle of the xylitol crystal particles is 65%, and the most suitable low humidity condition is 30%.

The equivalent particle radius of the crystal particle and the equivalent particle radius of the crystal particle to be predicted in the CHS crystal bridge growth model database are obtained by following operations:
weighing a mass of each particle size interval of the crystal particles after performing particle size screening on crystal particles to be measured, obtaining data $m_1, m_2, ..., m_n$, sequentially, counting a count of crystal particles for each particle size interval, obtaining data $P_1, P_2, ..., P_n$, sequentially, and calculating an equivalent particle radius $R_e$ of the crystal particle to be predicted according to following equations (e) and (f):

$$P_1 R_1{}^3 + P_2 R_2{}^3 + \cdots + P_n R_n{}^3 = (P_1 + P_2 + \cdots + P_n) R_e{}^3 , \qquad \text{equation (e)};$$

$$P_1 R_1{}^3 : P_2 R_2{}^3 : ... : P_n R_n{}^3 = m_1 : m_2 : ... : m_n , \qquad \text{equation (f)}$$

Specifically, the critical agglomeration cycle of the crystal particles with different equivalent particle radiuses under the multiple ambient temperatures and multiple environmental high and low humidity cycle conditions is obtained by:
loading a crystal particle to be measured into an agglomeration mold and placing the agglomeration mold in a box with set temperature, setting a high humidity and low humidity in a high and low humidity cycle within the both being the same with a high and low humidity cycle of the crystal particles to be measured, wherein a period of the high and low humidity cycle is 12-24 hours; counting a time of the high and low humidity cycle, and performing a de-molding operation for an agglomerate in the agglomeration mold; during the de-molding operation, in response to the agglomerate falls apart, determining that the agglomerate of the crystal particle to be measured is not agglomerated, and continuing to perform above operations to a next cycle until the agglomerate of the crystal particle does not fall apart, and recording a count of cycles completed to prevent the agglomeration from falling apart, and determining the count of cycles as a critical agglomeration cycle of the crystal particle to be measured under a corresponding ambient temperature and environmental high and low humidity cycle condition.

The box is a constant temperature and humidity box during actual use.

The processes above is not only suitable for determining the critical agglomeration cycle in the CHS crystal bridge growth model database, but also for verifying the predicted critical agglomeration cycle.

When determining the ritical agglomeration cycle, if the critical agglomeration cycle repeatedly determines for three times, calculating an average of the reference critical agglomeration cycles determined by three times as a critical agglomeration cycle after averaging treatment.

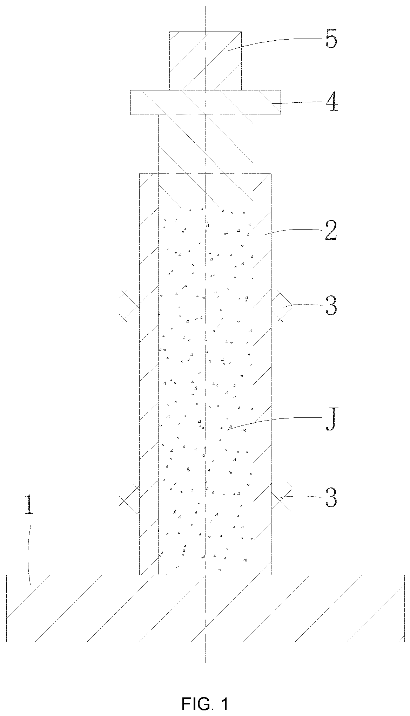

Specifically, FIG. 1 is a schematic diagram illustrating a structure profile of an agglomeration mold used for determining a critical agglomeration cycle according to some embodiments of the present disclosure. The agglomeration mold may include a base 1, a cylindrical container placed vertically on the base 1, a positioning sleeve 3 set around container 2 which is easy to disassemble, the container 2 may include a left housing and a right housing which is easy to demold, and the positioning sleeve 3 may fit the left housing and right housiniig together to form the container 2. A crystal particle product J to be measured is filled within the container 2, and multiple breathable small holes may be arranged on a side wall of the container 2 (not shown in the figure). An inner diameter of each breathable small hole may be smaller than a particle size of the crystal particle. A top of the crystal particle product may be provided with a presssing block 4, and a top of the pressing block 4 may be provided with a gravity weight 5. The pressing block 4 and the gravity weight 5 may simultaneously compress and compact the crystal particles in the container 2, making the crystal particles an agglomerate. When performing demolding operations on the agglomerate in the container 2, it is important to avoid applying additional stress to the agglomerate, which can cause the agglomerate to break and scatter, leading to misjudgment of the agglomeration cycle.

$V_{RH1}$, $V_{RH2}$, and $V_{RH3}$ in the equation (c) and equation (d) are calculated by:

under a same ambient temperature, a same environmental humidity, and different equivalent particle radiuses, calculating a moisture absorption capacity $V_{RH}'$ of a crystal particle with an equivalent particle radius $R_e'$ under an environmental humidity condition RH based on following equation (g):

$$\frac{V_{RH}}{V_{RH}'} = \frac{R_e}{R_e'}, \text{ equation (g)}$$

wherein $V_{RH}$ represents a moisture absorption capacity of existed crystal particles with the equivalent particle radius $R_e$ under the environmental humidity condition RH in the CHS crystal bridge growth model database, and $V_{RH}'$ is one of $V_{RH1}$, $V_{RH2}$, and $V_{RH3}$.

A determination of the moisture absorption capacity is performed by a dynamic steam adsorption instrument, before the determination, performing an initial drying treatment for the crystal particle to be measured in advance, and a mass of the crystal particle to be measured does not exceed 10 mg.

The following further illustrates the method for predicting a critical agglomeration cycl of a crystal particle in the present disclosure through specific embodiments.

Embodiment 1

A first embodiment of the method for predicting a critical agglomeration cycle of a crystal particle provided in the present disclosure includes following operations:

A certain quality of xylitol crystal particle products may be taked, a metallic screen ranging from 500 microns to 600 microns may be selected based on the particle size and particle distribution characteristics of the xylitol crystal particle products for measuring a mass of each particle size interval and counting a count of crystal particles in each particle size interval, and the equivalent particle radius $R_e$ may be calculated to be 550 microns according to the equations (e) and (f). Moisture absorption characteristics of the crystal particle products may be measured by a dynamic steam adsorption instrument to obtain a moisture absorption characteristic file of the crystal particle products at a temperature of 25 °C as shown in FIG. 2(a). as shown in FIG. 2(a), a deliquescence point of the xylitol crystal particle may be around 75%. Therefore, the high humidity condition for a critical agglomeration cycle experiment of the xylitol crystal particle may be set below 75%. The embodiments takes an equivalent particle radius of 550 microns, temperature of 25 °C, and a stored environmental high and low humidity cycle condition of 65%~30% as an example to illustrate how to obtain and predict other equivalent particle radiuses, such as critical agglomeration cycles of crystal particles of 312 microns, 404 microns, 507 microns, and 550 microns under different environmental high and low humidity cycle condition such as 65%~30%, 60%~30%, 55%~30%, and 50%~30%, and the critical agglomeration cycles may be compared with measured results of the critical agglomeration cycle to demonstrate the prediction accuracy of the process of the present disclosure.

A prediction process of the critical agglomeration cycle in the embodiments includes: Firstly, a dynamic vapor adsorption instrument may be used to measure a moisture absorption capacity $V_{RH}$ of an equivalent particle radius of 550 microns under different humidity conditions. The crystal particles may be sieved through a metal sieve ranging from 500 microns to 600 microns. As shown in FIG. 2(a), at 30% humidity condition, the moisture absorption $V_{RH}$ is 0.02000%, at 50% humidity condition, the moisture absorption $V_{RH}$ is 0.02740%, at 55% humidity condition, the moisture absorption $V_{RH}$ is 0.03266%, at 60% humidity condition, the moisture absorption $V_{RH}$ is 0.03747%, and at 65% humidity condition, the moisture absorption $V_{RH}$ is 0.04595%. Subsequently, the crystal particle product may be loaded into an agglomeration mold and the agglomeration mold may be placed in the constant temperature and humidity box, an humidity condition of the constant temperature and humidity box may be set to 65%~30% alternating high and low humidity cycle, and a critical agglomeration cycle N may be determined to be 7 times.

Then, a calculation eaquation may be used to calculate and predict the critical agglomeration cycle. The obtained parameters may be input into the CHS crystal bridge growth model database, according to an equation (g): $\dfrac{V_{RH}}{V_{RH}'} = \dfrac{R_e}{R_e'}$, moisture absorption capacities (i.e. a H-S file) of crystal particle products with different equivalent particle radiuses under the same humidity condition, calculation results may be shown in Table 1 below. For example, when a crystal particle with an equivalent particle radius of 312 microns is at 30% humidity, the moisture absorption capacity $V_{RH}$ is 0.01135%, when a crystal particle with an equivalent particle radius of 312 microns is at 50% humidity, the moisture absorption capacity $V_{RH}$ is 0.01554%, when a crystal particle with an equivalent particle radius of 312 microns is at 55% humidity, the moisture absorption capacity $V_{RH}$ is 0.01853%, when a crystal particle with an equivalent particle radius of 312 microns is at 60% humidity, the moisture absorption capacity $V_{RH}$ is 0.02126%, when a crystal particle with an equivalent particle radius of 312 microns is at 65% humidity, the moisture absorption capacity $V_{RH}$ is 0.02607%. Calculation results of the moisture absorption capacities of other equivalent particle radiuses, such as crystal particles of 404 microns or 507 microns under different humidity environments may be shown in Table 1.

Table 1 moisture absorption capacities of crystal particles with different equivalent particle radiuses

| $R_e$/(microns) | $V_{RH\text{-}30\%}$ | $V_{RH\text{-}50\%}$ | $V_{RH\text{-}55\%}$ | $V_{RH\text{-}60\%}$ | $V_{RH\text{-}65\%}$ |
|---|---|---|---|---|---|
| 550 | 0.02000 | 0.02740 | 0.03266 | 0.03747 | 0.04595 |
| 312 | 0.01135 | 0.01554 | 0.01853 | 0.02126 | 0.02607 |
| 404 | 0.01469 | 0.02013 | 0.02399 | 0.02752 | 0.03375 |
| 507 | 0.01844 | 0.02526 | 0.03011 | 0.03454 | 0.04236 |

In addition, it is now known that a critical agglomeration cycle $N_1$ of crystal particles with an equivalent particle radius $R_{1e}$ of 550 microns under a environmental high and low humidity cycle condition of 65%~30% is 7 times, obtained paramters may be input into the CHS crystal bridge growth model database, according to the equation (a): $\dfrac{N_1}{N_1'} = \left(\dfrac{R_{1e}}{R_{1e}'}\right)^4$, a critical agglomeration cycle $N_1'$ ((i.e., a C-S file) corresponding to crystal particles with an equivalent particle radius of $R_{1e}'$ under the same humidity RH condition may be calculated, and the critical agglomeration cycle may be taked as an integer, for example, a critical agglomeration cycle $N_1'$ of particle with an equivalent particle radius of 312 microns under a humidity cycle condition of 65%~30% is once, a critical agglomeration cycle $N_1'$ of particle with an equivalent particle radius of 404 microns under the humidity cycle condition of 65%~30% is twice, and a critical agglomeration cycle $N_1'$ of particle with an equivalent particle radius of 507 micons under the humidity cycle condition of 65%~30% is 5 times.

According to the H-S file and the eaquation (c): $V_I = V_{RH2} - V_{RH1}$, a moisture absorption capacity difference $V_I$ of crystal particles under single environmental high and low humidity cycle condition may be caclulated, and a moisture absorption capacity difference $V_I$ of crystal particles with an equivalent particle radius of 550 microns may be calculated. Similarly, moisture absorption capacity differences $V_I$ of crystal particles with other equivalent particle radiuses under different environmental high and low humidity cycle conditions may be obtained, and calculation results are shown in Table 2.

Table 2 moisture absorption capacity differences $V_l$ of crystal particles with different equivalent particle radiuses under an environmental high and low humidity cycle condition

| $R_e$/( microns) | $V_l$(50%-30%) | $V_l$(55%-30%) | $V_l$(60%-30%) | $V_l$(65%-30%) |
|---|---|---|---|---|
| 550 | 0.00740 | 0.01266 | 0.01747 | 0.02595 |
| 312 | 0.00419 | 0.00718 | 0.00991 | 0.01472 |
| 404 | 0.00544 | 0.00930 | 0.01283 | 0.01906 |
| 507 | 0.00682 | 0.01167 | 0.01610 | 0.02392 |

The moisture absorption capacity difference $V_l$ and the C-S file of crystal particles with different equivalent particle radiuses under the environmental high and low humidity cycling condition may be integrated, according to formula (b): $\frac{N_2}{N_2''} = \frac{V_l'}{V_l}$ , a critical agglomeration cycle $N_2''$ (i.e. a C-H file) may be calculated for the crystal particle products with the same equivalent particle radius under different environmental high and low humidity cycle conditions, and the critical agglomeration cycle may be taked as an integer, and calculation results are shown in Table 3. For example, a moisture absorption capacity difference $V_l$ (65% -30%) of crystal particles with an equivalent particle radius of 550 microns is 0.02595, and the critical agglomeration cycle $N_2$ under a environmental high and low humidity cycle condition of 65% -30% is 7 times, according to the Table 2, data of $V_l'$(60% -30%) is 0.01747, according to the equation (b), a critical agglomeration cycle $N_2''$ of crystal particles under a environmental high and low humidity cycle condition of 60% -30% is calculated to be 10 times. Similarly, critical agglomeration cycles $N_2''$ under other conditions may be calculated.

Table 3 predicted results of critical agglomeration cycles $N_2''$ of crystal particles with different equivalent particle radiuses under different environmental high and low humidity cycles conditions

| $R_e$/( microns) | $N_2''$(50%-30%) | $N_2''$(55%-30%) | $N_2''$(60%-30%) | $N_2''$(65%-30%) |
|---|---|---|---|---|
| 550 | 24 | 15 | 10 | 7 |
| 312 | 4 | 3 | 2 | 1 |
| 404 | 8 | 5 | 3 | 2 |
| 507 | 17 | 11 | 7 | 5 |

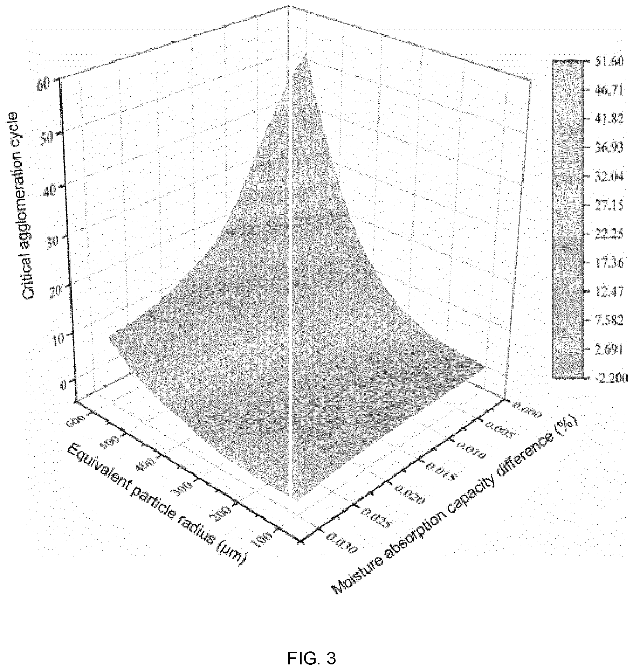

By integrating the data from the above files, a fitting diagram of predicted results of critical agglomeration cycles of xylitol multi-particle crystal particle products under different environmental high and low humidity cyclic storage conditions may be shown as shown in FIG. 3.

Next, the measured results of the critical agglomeration cycle may be verified with the predicted values. A certain quality of xylitol crystal particle products may be taken, and based on the particle size and distribution characteristics of the crystal particle products, select metal sieves of 200 μm-300 μm, 300 μm-400 μm, and 400 μm-500 μm for screening, median radius values corresponding to the particle size intervals are 250 microns, 350 microns, and 450 microns, respectively. After sieving, the mass of crystal particles in each particle size interval may be weighed, and a mass ratio may be approximately 1:1:1, and the equivalent particle radius $R_e$ may be calculated from the mass ratio of crystal particles in each particle size interval, which is 312 microns. Then, the dynamic vapor adsorption instrument may be used to measure the moisture absorption capacity of the crystal particle products at humidity of 50%, 55%, 60%, and 65%, respectively. The critical agglomeration cycle experiment may be conducted to set the environmental high and low humidity cycle conditions to 65%-30%, 60%-30%, 55%-30%, and 50%-30%, respectively, to determine the critical agglomeration cycle. The measured critical agglomeration cycle may be recorded. Similarly, crystal particles with equivalent particle radiuses of 404 microns and 507 microns may be screened separately, and the dynamic vapor adsorption instrument may be used to measure the moisture absorption capacity of the crystal particle products at humidity of 50%, 55%, 60%, and 65%, respectively. The critical agglomeration cycle experiment may be conducted to set the environmental high and low humidity cycle conditions to 65% -30%, 60% -30%, 55% -30%, and 50% -30%, respectively, to determine the critical agglomeration cycle.

As shown in FIG. 2(b), the moisture absorption capacity of crystal particles may increase linearly with equivalent particle radiuses of 312 microns, 404 microns, 507 microns, and 550 microns at humidity of 50%, 55%, 60%, and 65%, respectively, which can fit well with the predicted result according to the equation (g): $\frac{V_{RH}}{V_{RH}'} = \frac{R_e}{R_e'}$ , under humidity conditions of 50%, 55%, 60%, and 65%, fitted correlation coefficients may be 0.9966, 0.9896, 0.9982, and 0.9974, respectively, which is not less than 0.98, indicating a good fitting effect.

As shown in FIG. 2(c), an inverse proportional relationship is between the moisture absorption capacity and the critical agglomeration cycle, which has good consistency with the predicted result of the equation (b): $\frac{N_2}{N_2''} = \frac{V_l'}{V_l}$, fitted correlation coefficients of equivalent particle sizes of 311$\mu$m, 404$\mu$m, 507$\mu$m, and 550$\mu$m may be 0.9052, 0.9703, 0.9619, and 0.9578, which is not less than 0.90, indicating a good fitting effect.

As shown in FIG. 2(d), a fourth power function relationship is between the crystal particle size and the critical agglomeration cycle, which can fit well with the predicted result according to the equation (a): $\frac{N_1}{N_1'} = \left(\frac{R_{1e}}{R_{1e}'}\right)^4$. Under high humidity cycle conditions of 50%, 55%, 60%, and 65%, tted correlation coefficients may be 0.9767, 0.9679, 0.9035, and 0.9011, which is not less than 0.90, indicating a good fitting effect.

Therefore, the present disclosure uses the CHS crystal bridge growth model database to predict the critical agglomeration cycle of multi-particle crystal product under different environmental high and low humidity cycle conditions has a relatively accurate prediction effect, small experimental quantity, and fast prediction speed.

## Claims

1. A method for predicting a critical agglomeration cycle of a crystal particle, wherein the method is executed by a processor, wherein the method comprises:

   establishing a CHS crystal bridge growth model database of a crystal particle with a same type of a crystal particle to be predicted firstly, wherein the CHS crystal bridge growth model database includes: data of equivalent particle radius corresponding to different particle size standards of the same type of crystal particle, data of a moisture absorption capacity of crystal particles with different equivalent particle radiuses under multiple ambient temperatures and multiple ambient humidity conditions, respectively, and data of critical agglomeration cycles of the crystal particles with different equivalent particle radiuses under the multiple ambient temperatures and multiple environmental high and low humidity cycle conditions, respectively; then selecting existed data in the corresponding CHS crystal bridge growth model database based on an equivalent particle radius, a stored ambient temperature, and an environmental high and low humidity cycle condition of the crystal particle to be predicted, respectively, and calculating the critical agglomeration cycle according to following calculation equations, wherein a result obtained by calculation is a predicted critical agglomeration cycle of the crystal particle to be predicted;
   the calculation equations include:

   (1) in response to an ambient temperature and an environmental high and low humidity cycle condition of the crystal particle to be predicted being the same with data in the CHS crystal bridge growth model database and merely the equivalent particle radius of the crystal particle to be predicted being different from data in the CHS crystal bridge growth model database, a critical agglomeration cycle $N_1'$ of the crystal particle to be predicted under the ambient temperature and the environmental high and low humidity cycle condition according to equation (a):

$$\frac{N_1}{N_1'} = \left(\frac{R_{1e}}{R_{1e}'}\right)^4, \text{ equation (a)}$$

   in the above equation, $R_{1e}$ represents an equivalent particle radius of an existed crystal particle in the CHS crystal bridge growth model database, $R_{1e}'$ represents the equivalent particle radius of the crystal particle to be predicted, $N_1$ represents a critical agglomeration cycle of existed crystal particles with equivalent particle radius $R_{1e}$ in the CHS crystal bridge growth model under the ambient temperature and the environmental high and low humidity cycle condition, $N_1'$ represents the critical agglomeration cycle of the crystal particle to be predicted, and $N_1$ and $N_1'$ are an integer not less than 1, respectively;
   (2) in response to the ambient temperature and the equivalent particle radius of the crystal particle to be predicted are the same with the data in the CHS crystal bridge growth model database and merely the environmental high and low humidity cycle condition of the crystal particle to be predicted being different from data in the CHS crystal bridge growth model database, a critical agglomeration cycle $N_2''$ of the crystal particle to be predicted under the ambient temperature and the environmental high and low humidity cycle condition according to equation (b):

$$\frac{N_2}{N_2''} = \frac{V_l'}{V_l}, \text{ equation (b)}$$

wherein,

$$V_l = V_{RH2} - V_{RH1}, \text{ equation (c)};$$

$$V_l' = V_{RH3} - V_{RH1}, \text{ equation (d)};$$

in the above equations, $V_{RH2}$ represents a moisture absorption capacity of the existed crystal particle in the CHS crystal bridge growth model database under a high humidity condition in a first environmental high and low humidity cycle; $V_{RH3}$ represents a moisture absorption capacity of the existed crystal particle in the CHS crystal bridge growth model database under a high humidity condition in a second environmental high and low humidity cycle, wherein a low humidity condition in the first environmental high and low humidity cycle is the same with a low humidity condition in the second environmental high and low humidity cycle; $V_{RH1}$ represents a moisture absorption capacity of the existed crystal particle in the CHS crystal bridge growth model database under a low humidity condition in the first environmental high and low humidity cycle; $V_l$ represents a moisture absorption capacity difference between the high humidity condition and the low humidity condition in the first environmental high and low humidity cycle; $V_l'$ represents a moisture absorption capacity difference between the high humidity condition and the low humidity condition in the second environmental high and low humidity cycle; $N_2$ represents a critical agglomeration cycle of the existed crystal particle in the CHS crystal bridge growth model database under the high humidity condition and the low humidity condition in the first environmental high and low humidity cycle; $N_2''$ represents a critical agglomeration cycle of the crystal particle to be predicted under the high humidity condition and the low humidity condition in the second environmental high and low humidity cycle, and $N_2$ and $N_2''$ are an integer not less than 1, respectively;

wherein the high humidity condition in the environmental high and low humidity cycle is lower than a deliquescence point of the same type of crystal particles, such that the critical agglomeration cycle is greater than once when a particle size of the crystal particle is less than 100 microns, the low humidity condition is less than the high humidity condition, and a humidity difference between the low humidity condition and the high humidity condition is greater than 20%,

wherein the critical agglomeration cycle of the crystal particles with different equivalent particle radiuses under the multiple ambient temperatures and multiple environmental high and low humidity cycle conditions is obtained by:

loading a crystal particle to be measured into an agglomeration mold and placing the agglomeration mold in a box with set temperature, setting a high humidity and low humidity in a high and low humidity cycle within the box being the same with a high and low humidity cycle of the crystal particles to be measured, wherein a period of the high and low humidity cycle is 12-24 hours; counting a time of the high and low humidity cycle, and performing a de-molding operation for an agglomerate in the agglomeration mold;

during the de-molding operation, in response to the agglomerate falls apart, determining that the agglomerate of the crystal particle to be measured is not agglomerated, and continuing to perform above operations to a next cycle until the agglomerate of the crystal particle does not fall apart, and recording a count of cycles completed to prevent the agglomeration from falling apart, and determining the count of cycles as a critical agglomeration cycle of the crystal particle to be measured under a corresponding ambient temperature and environmental high and low humidity cycle condition.

2. The method for predicting the critical agglomeration cycle of the crystal particle of claim 1, wherein the equivalent particle radius of the crystal particle and the equivalent particle radius of the crystal particle to be predicted in the CHS crystal bridge growth model database are obtained by following operations:

weighing a mass of each particle size interval of the crystal particles after performing particle size screening on crystal particles to be measured, obtaining data $m_1, m_2, ..., m_n$, sequentially, counting a count of crystal particles for each particle size interval, obtaining data $P_1, P_2, ..., P_n$, sequentially, and calculating an equivalent particle radius $R_e$ of the crystal particles to be measured according to following equations, the equations include:

$$P_1 R_1{}^3 + P_2 R_2{}^3 + \cdots + P_n R_n{}^3 = (P_1 + P_2 + \cdots + P_n) R_e{}^3 \ , \qquad \text{equation (e)};$$

$$P_1 R_1{}^3 : P_2 R_2{}^3 : \ldots : P_n R_n{}^3 = m_1 : m_2 : \ldots : m_n \ , \qquad \text{equation (f)}$$

3. The method for predicting the critical agglomeration cycle of the crystal particle of claim 1, when determining the critical agglomeration cycle, if the critical agglomeration cycle repeatedly determines for three times, calculating an average of the reference critical agglomeration cycles determined by three times as a critical agglomeration cycle after averaging treatment.

4. The method for predicting the critical agglomeration cycle of the crystal particle of claim 1, wherein $V_{RH1}$, $V_{RH2}$, and $V_{RH3}$ in the equation (c) and equation (d) are calculated by:

under a same ambient temperature, a same environmental humidity, and different equivalent particle radiuses, calculating a moisture absorption capacity $V_{RH}'$ of a crystal particle with an equivalent particle radius $R_e'$ under an environmental humidity condition RH based on following equation (g):

$$\frac{V_{RH}}{V_{RH}'} = \frac{R_e}{R_e'}, \ \text{equation (g)}$$

wherein $V_{RH}$ represents a moisture absorption capacity of existed crystal particles with the equivalent particle radius $R_e$ under the environmental humidity condition RH in the CHS crystal bridge growth model database, and $V_{RH}'$ is one of $V_{RH1}$, $V_{RH2}$, and VRH3.

5. The method for predicting the critical agglomeration cycle of the crystal particle of claim 1, wherein a determination of the moisture absorption capacity is performed by a dynamic steam adsorption instrument, before the determination, performing an initial drying treatment for the crystal particle to be measured in advance, and a mass of the crystal particle to be measured does not exceed 10 mg.

6. The method for predicting the critical agglomeration cycle of the crystal particle of claim 1, wherein a high humidity condition of the environmental high and low humidity cycle for a xylitol crystal particle is 65% and a low humidity condition of the environmental high and low humidity cycle for the xylitol crystal particle is 30%.

**Patentansprüche**

1. Verfahren zum Vorhersagen eines kritischen Agglomerationszyklus eines Kristallpartikels, wobei das Verfahren von einem Prozessor ausgeführt wird, wobei das Verfahren Folgendes umfasst:

Erstellen einer CHS-Kristallbrücken-Wachstumsmodelldatenbank eines Kristallpartikels, wobei ein selber Typ eines Kristallpartikels zuerst vorhergesagt werden soll, wobei die CHS-Kristallbrücken-Wachstumsmodelldatenbank Folgendes beinhaltet: Daten gleichwertiger Partikelradien, entsprechend unterschiedlichen Partikelgrößenstandards desselben Typs von Kristallpartikel, Daten einer Feuchtigkeitsabsorptionskapazität von Kristallpartikeln mit unterschiedlichen gleichwertigen Partikelradien unter mehreren Umgebungstemperaturen beziehungsweise mehreren Umgebungsfeuchtigkeitsbedingungen, und Daten kritischer Agglomerationszyklen der Kristallpartikel mit unterschiedlichen gleichwertigen Partikelradien unter den mehreren Umgebungstemperaturen beziehungsweise mehreren Zyklusbedingungen hoher und niedriger Umgebungsfeuchtigkeit; dann Auswählen bestehender Daten in der entsprechenden CHS-Kristallbrücken-Wachstumsmodelldatenbank auf Basis eines gleichwertigen Partikelradius, einer gespeicherten Umgebungstemperatur beziehungsweise einer Zyklusbedingung hoher und niedriger Umgebungsfeuchtigkeit des Kristallpartikels, das vorhergesagt werden soll, und Berechnen des kritischen Agglomerationszyklus gemäß folgenden Berechnungsgleichungen, wobei ein Ergebnis, das durch Berechnung erhalten wird, ein vorhergesagter kritischer Agglomerationszyklus des Kristallpartikels ist, das vorhergesagt werden soll;
die Berechnungsgleichung beinhaltet:

(1) infolgedessen, dass eine Umgebungstemperatur und eine Zyklusbedingung hoher und niedriger Umgebungsfeuchtigkeit des Kristallpartikels, das vorhergesagt werden soll, mit Daten in der CHS-Kristallbrücken-Wachstumsmodelldatenbank übereinstimmen und sich bloß der gleichwertige Partikelradius des Kristallpartikels, das vorhergesagt werden soll, von Daten in der CHS-Kristallbrücken-Wachstumsmodelldatenbank unterscheidet, einen kritischen Agglomerationszyklus $N_1'$ des Kristallpartikels, das vorhergesagt werden soll, unter der Umgebungstemperatur und der Zyklusbedingung hoher und niedriger Umgebungsfeuchtigkeit gemäß Gleichung (a):

$$\frac{N_1}{N_1{}'} = \left(\frac{R_{1e}}{R_{1e}{}'}\right)^4, \text{ Gleichung (a)}$$

wobei in der vorstehenden Gleichung $R_{1e}$ für einen gleichwertigen Partikelradius eines bestehenden Kristallpartikels in der CHS-Kristallbrücken-Wachstumsmodelldatenbank steht, $R_{1e}'$ für den gleichwertigen Partikelradius des Kristallpartikels steht, das vorhergesagt werden soll, $N_1$ für einen kritischen Agglomerationszyklus bestehender Kristallpartikel mit gleichwertigem Partikelradius $R_{1e}$ in dem CHS-Kristallbrücken-Wachstumsmodell unter der Umgebungstemperatur und der Zyklusbedingung hoher und niedriger Luftfeuchtigkeit steht, $N_1'$ für den kritischen Agglomerationszyklus des Kristallpartikels steht, das vorhergesagt werden soll, und $N_1$ beziehungsweise $N_1'$ ganze Zahlen nicht kleiner als 1 sind;

(2) infolgedessen, dass die Umgebungstemperatur und der gleichwertige Partikelradius des Kristallpartikels, das vorhergesagt werden soll, mit den Daten in der CHS-Kristallbrücken-Wachstumsmodelldatenbank übereinstimmen und bloß die Zyklusbedingung hoher und niedriger Umgebungsfeuchtigkeit des Kristallpartikels, das vorhergesagt werden soll, sich von Daten in der CHS-Kristallbrücken-Wachstumsmodelldatenbank unterscheidet, einen kritischen Agglomerationszyklus $N_2''$ des Kristallpartikels, das vorhergesagt werden soll, unter der Umgebungstemperatur und der Zyklusbedingung hoher und niedriger Umgebungsfeuchtigkeit gemäß Gleichung (b):

$$\frac{N_2}{N_2{}''} = \frac{V_l{}'}{V_l}, \text{ Gleichung (b)}$$

wobei

$$V_l = V_{RH2} - V_{RH1}, \text{ Gleichung (c);}$$

$$V_l{}' = V_{RH3} - V_{RH1}, \text{ Gleichung (d);}$$

wobei in den vorstehenden Gleichungen $V_{RH2}$ für eine Feuchtigkeitsabsorptionskapazität des bestehenden Kristallpartikels in der CHS-Kristallbrücken-Wachstumsmodelldatenbank unter einer Bedingung hoher Feuchtigkeit in einem ersten Zyklus hoher und niedriger Umgebungsfeuchtigkeit steht; $V_{RH3}$ für eine Feuchtigkeitsabsorptionskapazität des bestehenden Kristallpartikels in der CHS-Kristallbrücken-Wachstumsmodelldatenbank unter einer Bedingung hoher Feuchtigkeit in einem zweiten Zyklus hoher und niedriger Umgebungsfeuchtigkeit steht, wobei eine Bedingung niedriger Feuchtigkeit in dem ersten Zyklus hoher und niedriger Umgebungsfeuchtigkeit mit einer Bedingung niedriger Feuchtigkeit in dem zweiten Zyklus hoher und niedriger Umgebungsfeuchtigkeit übereinstimmt; $V_{RH1}$ für eine Feuchtigkeitsabsorptionskapazität des bestehenden Kristallpartikels in der CHS-Kristallbrücken-Wachstumsmodelldatenbank unter einer Bedingung niedriger Feuchtigkeit in dem ersten Zyklus hoher und niedriger Umgebungsfeuchtigkeit steht; $V_l$ für eine Feuchtigkeitsabsorptionskapazitätsdifferenz zwischen der Bedingung hoher Feuchtigkeit und der Bedingung niedriger Feuchtigkeit in dem ersten Zyklus hoher und niedriger Umgebungsfeuchtigkeit steht; $V_l'$ für eine Feuchtigkeitsabsorptionskapazitätsdifferenz zwischen der Bedingung hoher Feuchtigkeit und der Bedingung niedriger Feuchtigkeit in dem zweiten Zyklus hoher und niedriger Umgebungsfeuchtigkeit steht; $N_2$ für einen kritischen Agglomerationszyklus des bestehenden Kristallpartikels in der CHS-Kristallbrücken-Wachstumsmodelldatenbank unter der Bedingung hoher Feuchtigkeit und der Bedingung niedriger Feuchtigkeit in dem ersten Zyklus hoher und niedriger Umgebungsfeuchtigkeit steht; $N_2''$ für einen kritischen Agglomerationszyklus des Kristallpartikels, das vorhergesagt werden soll, unter der Bedingung hoher Feuchtigkeit und der Bedingung niedriger Feuchtigkeit in dem zweiten Zyklus hoher und niedriger Umgebungsfeuchtigkeit steht, und $N_2$ beziehungsweise $N_2''$ eine ganze Zahl nicht kleiner als 1 sind;

wobei die Bedingung hoher Feuchtigkeit in dem Zyklus hoher und niedriger Umgebungsfeuchtigkeit geringer als ein Deliqueszenzpunkt desselben Typs von Kristallpartikeln ist, sodass der kritische Agglomerationszyklus größer als eins ist, wenn eine Partikelgröße des Kristallpartikels kleiner als 100 Mikrometer ist, die Bedingung niedriger Feuchtigkeit geringer als die Bedingung hoher Feuchtigkeit ist und eine Feuchtigkeitsdifferenz zwischen der Bedingung niedriger Feuchtigkeit und der Bedingung hoher Feuchtigkeit größer als 20% ist, wobei der kritische Agglomerationszyklus der Kristallpartikel mit unterschiedlichen gleichwertigen Partikelradien unter den mehreren Umgebungstemperaturen und mehreren Zyklusbedingungen hoher und niedriger Umgebungsfeuchtigkeit durch Folgendes erhalten wird:

Laden eines Kristallpartikels, das gemessen werden soll, in eine Agglomerationsform und Platzieren der Agglomerationsform in einem Kasten mit festgelegter Temperatur, Einstellen einer hohen Feuchtigkeit und niedrigen Feuchtigkeit in einem Zyklus hoher und niedriger Feuchtigkeit innerhalb des Kastens, der mit einem Zyklus hoher und niedriger Feuchtigkeit der Kristallpartikel übereinstimmt, die gemessen werden sollen, wobei eine Periode des Zyklus hoher und niedriger Feuchtigkeit 12-24 Stunden beträgt; Zählen einer Zeit des Zyklus hoher und niedriger Feuchtigkeit und Durchführen eines Entformungsbetriebs für ein Aggregat in der Agglomerationsform; während des Entformungsbetriebs, infolgedessen, dass das Aggregat zerfällt, Bestimmen, dass das Aggregat des Kristallpartikels, das gemessen werden soll, nicht aggregiert wurde, und Fortsetzen, vorstehende Betriebe bis zu einem nächsten Zyklus durchzuführen, bis das Aggregat des Kristallpartikels nicht zerfällt, und Aufzeichnen einer Anzahl von Zyklen, die abgeschlossen worden sind, um ein Zerfallen des Aggregats zu verhindern, und Bestimmen der Anzahl von Zyklen als einen kritischen Agglomerationszyklus des Kristallpartikels, das gemessen werden soll, unter einer entsprechenden Umgebungstemperatur und Zyklusbedingung hoher und niedriger Umgebungsfeuchtigkeit.

2. Verfahren zum Vorhersagen des kritischen Agglomerationszyklus des Kristallpartikels nach Anspruch 1, wobei der gleichwertige Partikelradius des Kristallpartikels und der gleichwertige Partikelradius des Kristallpartikels, das vorhergesagt werden soll, in der CHS-Kristallbrücken-Wachstumsmodelldatenbank durch folgende Operationen erhalten werden:
Wiegen einer Masse jedes Partikelgrößenintervalls der Kristallpartikel nach Durchführen von Partikelgrößenuntersuchung an Kristallpartikeln, die gemessen werden sollen, Erhalten von Daten $m_1$, $m_2$, ..., $m_n$ nacheinander, Zählen einer Zahl von Kristallpartikeln für jedes Partikelgrößenintervall, Erhalten von Daten $P_1$, $P_2$, ..., $P_n$ nacheinander, und Berechnen eines gleichwertigen Partikelradius $R_e$ der Kristallpartikel, die gemessen werden sollen, gemäß folgenden Gleichungen, wobei die Gleichungen beinhalten:

$$P_1R_1{}^3 + P_2R_2{}^3 + \cdots + P_nR_n{}^3 = (P_1+P_2+\cdots+P_n)R_e{}^3, \text{ Gleichung (e);}$$

$$P_1R_1{}^3 : P_2R_2{}^3 : \ldots : P_nR_n{}^3 = m_1 : m_2 : \ldots : m_n, \text{ Gleichung (f)}$$

3. Verfahren zum Vorhersagen des kritischen Agglomerationszyklus des Kristallpartikels nach Anspruch 1, falls beim Bestimmen des kritischen Agglomerationszyklus der kritische Agglomerationszyklus wiederholt dreimal bestimmt wird, Berechnen eines Durschnitts der kritischen Referenzagglomerationszyklen, die dreimal bestimmt wurden, als einen kritischen Agglomerationszyklus nach einer Mittelungsbehandlung.

4. Verfahren zum Vorhersagen des kritischen Agglomerationszyklus des Kristallpartikels nach Anspruch 1, wobei $V_{RH1}$, $V_{RH2}$ und $V_{RH3}$ in der Gleichung (c) und Gleichung (d) durch Folgendes berechnet werden:

unter einer selben Umgebungstemperatur, einer selben Umgebungsfeuchtigkeit und unterschiedlichen gleichwertigen Partikelradien, Berechnen einer Feuchtigkeitsabsorptionskapazität $V_{RH}{}'$ eines Kristallpartikels mit einem gleichwertigen Partikelradius $R_e{}'$ unter einer Umgebungsfeuchtigkeitsbedingung RH auf Basis folgender Gleichung (g):

$$\frac{V_{RH}}{V_{RH}{}'} = \frac{R_e}{R_e{}'}, \text{ Gleichung (g)}$$

wobei $V_{RH}$ für eine Feuchtigkeitsabsorptionskapazität bestehender Kristallpartikel mit dem gleichwertigen Partikelradius $R_e$ unter der Umgebungsfeuchtigkeitsbedingung RH in der CHS-Kristallbrücken-Wachstumsmodelldatenbank steht und $V_{RH}{}'$ eines von $V_{RH1}$, $V_{RH2}$ und $V_{RH3}$ ist.

**5.** Verfahren zum Vorhersagen des kritischen Agglomerationszyklus des Kristallpartikels nach Anspruch 1, wobei eine Bestimmung der Feuchtigkeitsabsorptionskapazität durch ein dynamisches Dampfadsorptionsinstrument durchgeführt wird, vor der Bestimmung im Voraus eine anfängliche Trocknungsbehandlung für das Kristallpartikel, das gemessen werden soll, durchgeführt wird und eine Masse des Kristallpartikels, das gemessen werden soll, 10 mg nicht übersteigt.

**6.** Verfahren zum Vorhersagen des kritischen Agglomerationszyklus des Kristallpartikels nach Anspruch 1, wobei eine hohe Feuchtigkeitsbedingung des Zyklus hoher und niedriger Umgebungsfeuchtigkeit für ein Xylitol-Kristallpartikel 65% ist und eine niedrige Feuchtigkeitsbedingung des Zyklus hoher und niedriger Umgebungsfeuchtigkeit für das Xylitol-Kristallpartikel 30% ist.

**Revendications**

**1.** Procédé de prédiction d'un cycle d'agglomération critique d'une particule cristalline, dans lequel le procédé est exécuté par un processeur, dans lequel le procédé comprend :

l'établissement d'une base de données de modèles de croissance de pont cristallin CHS d'une particule cristalline d'un type identique à celui d'une particule cristalline à prédire en premier, dans lequel la base de données de modèles de croissance de pont cristallin CHS inclut : des données de rayon de particule équivalent correspondant à différentes normes de taille de particule du même type de particule cristalline, des données d'une capacité d'absorption d'humidité de particules cristallines de différents rayons de particule équivalents respectivement à de multiples températures ambiantes et dans de multiples conditions d'humidité ambiante, et des données de cycles d'agglomération critique des particules cristallines de différents rayons de particule équivalents respectivement aux multiples températures ambiantes et dans de multiples conditions de cycles de forte humidité et de faible humidité d'environnement ; puis la sélection de données existantes dans la base de données de modèles de croissance de pont cristallin CHS correspondante sur la base respectivement d'un rayon de particule équivalent, d'une température ambiante stockée et d'une condition de cycle de forte humidité et de faible humidité d'environnement de la particule cristalline à prédire, et le calcul du cycle d'agglomération critique selon des équations de calcul suivantes, dans lequel un résultat obtenu par le calcul est un cycle d'agglomération critique prédit de la particule cristalline à prédire ;
les équations de calcul incluent :

(1) en réponse au fait qu'une température ambiante et qu'une condition de cycle de forte humidité et de faible humidité d'environnement de la particule cristalline à prédire sont identiques à des données dans la base de données de modèles de croissance de pont cristallin CHS et que seulement le rayon de particule équivalent de la particule cristalline à prédire est différent de données dans la base de données de modèles de croissance de pont cristallin CHS, un cycle d'agglomération critique $N_1'$ de la particule cristalline à prédire à la température ambiante et dans la condition de cycle de forte humidité et de faible humidité d'environnement est selon l'équation (a) :

$$\frac{N_1}{N_1'} = \left(\frac{R_{1e}}{R_{1e}'}\right)^4, \text{ équation (a)}$$

dans l'équation ci-dessus, $R_{1e}$ représente un rayon de particule équivalent d'une particule cristalline existante dans la base de données de modèles de croissance de pont cristallin CHS, $R_{1e}'$ représente le rayon de particule équivalent de la particule cristalline à prédire, $N_1$ représente un cycle d'agglomération critique de particules cristallines existantes d'un rayon de particule équivalent $R_{1e}$ dans la base de données de modèles de croissance de pont cristallin CHS à la température ambiante et dans la condition de cycle de forte humidité et de faible humidité d'environnement, $N_1'$ représente le cycle d'agglomération critique de la particule cristalline à prédire, et $N_1$ et $N_1'$ sont respectivement un entier pas inférieur à 1 ;
(2) en réponse au fait que la température ambiante et que le rayon de particule équivalent de la particule cristalline à prédire sont identiques à des données dans la base de données de modèles de croissance de pont cristallin CHS et que seulement la condition de cycle de forte humidité et de faible humidité d'environnement de la particule cristalline à prédire est différente de données dans la base de données de modèles

16

de croissance de pont cristallin CHS, un cycle d'agglomération critique $N_2''$ de la particule cristalline à prédire à la température ambiante et dans la condition de cycle de forte humidité et de faible humidité d'environnement est selon l'équation (b) :

$$\frac{N_2}{N_2''} = \frac{V_l'}{V_l}, \text{ équation (b)}$$

dans lequel,

$$V_l = V_{RH2} - V_{RH1}, \text{ équation (c) ;}$$

$$V_l' = V_{RH3} - V_{RH1}, \text{ équation (d) ;}$$

dans les équations ci-dessus, $V_{RH2}$ représente une capacité d'absorption d'humidité de la particule cristalline existante dans la base de données de modèles de croissance de pont cristallin CHS dans une condition de forte humidité dans un premier cycle de forte humidité et de faible humidité d'environnement ; $V_{RH3}$ représente une capacité d'absorption d'humidité de la particule cristalline existante dans la base de données de modèles de croissance de pont cristallin CHS dans une condition de forte humidité dans un deuxième cycle de forte humidité et de faible humidité d'environnement, dans lequel une condition de faible humidité dans le premier cycle de forte humidité et de faible humidité d'environnement est identique à une condition de faible humidité dans le deuxième cycle de forte humidité et de faible humidité d'environnement ; $V_{RH1}$ représente une capacité d'absorption d'humidité de la particule cristalline existante dans la base de données de modèles de croissance de pont cristallin CHS dans une condition de faible humidité dans le premier cycle de forte humidité et de faible humidité d'environnement ; $V_l$ représente une différence de capacité d'absorption d'humidité entre la condition de forte humidité et la condition de faible humidité dans le premier cycle de forte humidité et de faible humidité d'environnement ; $V_l'$ représente une différence de capacité d'absorption d'humidité entre la condition de forte humidité et la condition de faible humidité dans le deuxième cycle de forte humidité et de faible humidité d'environnement ; $N_2$ représente un cycle d'agglomération critique de la particule cristalline existante dans la base de données de modèles de croissance de pont cristallin CHS dans la condition de forte humidité et dans la condition de faible humidité dans le premier cycle de forte humidité et de faible humidité d'environnement ; $N_2''$ représente un cycle d'agglomération critique de la particule cristalline à prédire dans la condition de forte humidité et dans la condition de faible humidité dans le deuxième cycle de forte humidité et de faible humidité d'environnement, et $N_2$ et $N_2''$ sont respectivement un entier pas inférieur à 1 ;

dans lequel la condition de forte humidité dans le cycle de forte humidité et de faible humidité d'environnement est inférieure à un point de déliquescence du même type de particules cristallines, de sorte que le cycle d'agglomération critique soit supérieur à une fois lorsqu'une taille de particule de la particule cristalline est inférieure à 100 microns, la condition de faible humidité est inférieure à la condition de forte humidité, et une différence d'humidité entre la condition de faible humidité et la condition de forte humidité est supérieure à 20 %,

dans lequel le cycle d'agglomération critique des particules cristallines de rayons de particule équivalents différents aux multiples températures ambiantes et dans les multiples conditions de cycle de forte humidité et de faible humidité d'environnement est obtenu par :

le chargement d'une particule cristalline à mesurer dans un moule d'agglomération et le placement du moule d'agglomération dans une boîte à une température de consigne, la définition d'une forte humidité et d'une faible humidité dans un cycle de forte humidité et de faible humidité dans la boîte pour qu'elles soient identiques à celles dans un cycle de forte humidité et de faible humidité des particules cristallines à mesurer, dans lequel une période du cycle de forte humidité et de faible humidité est de 12 à 24 heures ; le décompte d'un temps du cycle de forte humidité et de faible humidité, et la réalisation d'une opération de démoulage pour un aggloméré dans le moule d'agglomération ;

pendant l'opération de démoulage, en réponse à la décomposition de l'aggloméré, la détermination que l'aggloméré de la particule cristalline à mesurer n'est pas aggloméré, et la poursuite de la réalisation des opérations ci-dessus dans un cycle suivant jusqu'à ce que l'aggloméré de la particule cristalline ne se décompose pas, et l'enregistrement d'un nombre de cycles effectués pour empêcher la décomposition de l'aggloméré, et la détermination du nombre de cycles en tant que cycle d'agglomération critique de la particule cristalline à mesurer à une température ambiante correspondante et dans des conditions de cycle de forte humidité et de faible humidité d'environnement correspondantes.

2. Procédé de prédiction du cycle d'agglomération critique de la particule cristalline selon la revendication 1, dans lequel le rayon de particule équivalent de la particule cristalline et le rayon de particule équivalent de la particule cristalline à prédire dans la base de données de modèles de croissance de pont cristallin CHS sont obtenus par les opérations suivantes :
le pesage d'une masse de chaque intervalle de taille de particule des particules cristallines après la réalisation d'un filtrage de taille de particule sur des particules cristallines à mesurer, l'obtention de données $m_1, m_2, ..., m_n$ de manière séquentielle, le décompte d'un nombre de particules cristallines pour chaque intervalle de taille de particule, l'obtention de données $P_1, P_2, ..., P_n$ de manière séquentielle, et le calcul d'un rayon de particule équivalent $R_e$ des particules cristallines à mesurer selon les équations suivantes, les équations incluant :

$$P_1 R_1^3 + P_2 R_2^3 + \cdots + P_n R_n^3 = (P_1 + P_2 + \cdots + P_n) R_e^3, \text{équation (e) ;}$$

$$P_1 R_1^3 : P_2 R_2^3 : ... : P_n R_n^3 = m_1 : m_2 : ... : m_n, \text{équation (f).}$$

3. Procédé de prédiction du cycle d'agglomération critique de la particule cristalline selon la revendication 1, comprenant en outre, lors de la détermination du cycle d'agglomération critique, si le cycle d'agglomération critique est déterminé trois fois de suite, le calcul d'une moyenne des cycles d'agglomération critique de référence déterminés trois fois en tant que cycle d'agglomération critique après un traitement de moyenne.

4. Procédé de prédiction du cycle d'agglomération critique de la particule cristalline selon la revendication 1, dans lequel $V_{RH1}$, $V_{RH2}$ et $V_{RH3}$ dans l'équation (c) et dans l'équation (d) sont calculés par :

à une même température ambiante, à une même humidité d'environnement et à des rayons de particule équivalents différents, le calcul d'une capacité d'absorption d'humidité $V'_{RH}$ d'une particule cristalline d'un rayon de particule équivalent $R'_e$ dans une condition d'humidité d'environnement **RH** selon l'équation (g) suivante :

$$\frac{V_{RH}}{V'_{RH}} = \frac{R_e}{R'_e}, \text{équation (g),}$$

dans lequel $V_{RH}$ représente une capacité d'absorption d'humidité de particules cristallines existantes de rayon de particule équivalent $R_e$ dans la condition d'humidité d'environnement **RH** dans la base de données de modèles de croissance de pont cristallin CHS, et $V'_{RH}$ est l'un parmi $V_{RH1}$, $V_{RH2}$, et $V_{RH3}$.

5. Procédé de prédiction du cycle d'agglomération critique de la particule cristalline selon la revendication 1, dans lequel une détermination de la capacité d'absorption d'humidité est réalisée par un instrument d'absorption de vapeur dynamique ; comprenant en outre, avant la détermination, la réalisation à l'avance d'un traitement de séchage initial de la particule cristalline à mesurer ; et dans lequel une masse de la particule cristalline à mesurer ne dépasse pas 10 mg.

6. Procédé de prédiction du cycle d'agglomération critique de la particule cristalline selon la revendication 1, dans lequel une condition de forte humidité du cycle de forte humidité et de faible humidité d'environnement pour une particule cristalline de xylitol est 65 % et une condition de faible humidité du cycle de forte humidité et de faible humidité d'environnement pour la particule cristalline de xylitol est 30 %.

FIG. 1

FIG. 2(a)

FIG. 2(b)

FIG. 2(c)

FIG. 2(d)

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108959817 A

**Non-patent literature cited in the description**

- **CHEN MINGYANG et al.** The time and location dependent prediction of crystal caking by a modified crystal bridge growth model and DEM simulation considering particle size and shape. *CHEMICAL ENGINEERING SCIENCE, OXFORD, GB*, 09 December 2019, vol. 214, ISSN 0009-2509